(19) **Europäisches Patentamt / European Patent Office / Office européen des brevets**

(11) **EP 4 752 768 A1**

(12) **EUROPEAN PATENT APPLICATION**

(43) Date of publication:
**03.06.2026 Bulletin 2026/23**

(21) Application number: **25219981.5**

(22) Date of filing: **02.12.2025**

(51) International Patent Classification (IPC):
$G06F\ 21/60^{(2013.01)}$    $G06F\ 21/62^{(2013.01)}$
$G06N\ 20/20^{(2019.01)}$    $G16H\ 50/20^{(2018.01)}$
$H04L\ 9/06^{(2006.01)}$    $H04L\ 9/08^{(2006.01)}$
$H04L\ 9/40^{(2022.01)}$    $G16H\ 10/60^{(2018.01)}$

(52) Cooperative Patent Classification (CPC):
**G06F 21/606; G06F 21/6245; G06N 20/20;
G16H 10/60; G16H 50/20; H04L 9/0637;
H04L 9/0861; H04L 9/0866; H04L 9/40;
H04L 63/0435; H04L 63/06; H04L 2209/88;
Y02T 10/40**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **02.12.2024 CN 202411746983**

(71) Applicants:
- **Peking Union Medical College Hospital, Chinese
  Academy of Medical Sciences & Peking Union
  Medical College**
  **Beijing 100730 (CN)**
- **Digital Health China Technologies Co., Ltd.**
  **Beijing 100080 (CN)**

(72) Inventors:
- **GUO, Jian**
  **Beijing, 100730 (CN)**
- **ZHANG, Shuyang**
  **Beijing, 100730 (CN)**

- **JIN, Ye**
  **Beijing, 100730 (CN)**
- **ZHANG, Wen**
  **Beijing, 100730 (CN)**
- **GONG, Mengchun**
  **Beijing, 100080 (CN)**
- **SHI, Wenzhao**
  **Beijing, 100080 (CN)**
- **ZHENG, Xihong**
  **Beijing, 100080 (CN)**
- **XIA, Peng**
  **Beijing, 100730 (CN)**
- **PENG, Linyi**
  **Beijing, 100730 (CN)**
- **LIU, Peng**
  **Beijing, 100730 (CN)**

(74) Representative: **Lapienis, Juozas**
  **MSP Europe UAB
  21-92 Seimyniskiu Str.
  09236 Vilnius (LT)**

(54) **ENCRYPTED TRANSMISSION SYSTEM AND METHOD FOR MEDICAL DATA**

(57) The present disclosure provides an encrypted transmission system and method for medical data, which relates to the technical field of medical data. A server, in response to a medical data analysis request, performs encrypted transmission of model parameters with each client by the following steps: the server receives a model parameter acquisition request sent by the client; the server performs identity authentication of the client based on request authentication information corresponding to the client; when the identity authentication of the client is passed, the server generates a key string based on historical optimized model parameters corresponding to the client and performs encryption on a current round of to-be sent optimized model parameters to generate a ciphertext data packet and send to the client; the client determines a decryption key based on the identifier and decrypts the received ciphertext data packet to obtain the optimized model parameters; and the client updates a local analysis model based on the obtained optimized model parameters, enabling the server to determine a next round of optimized model parameters for the client.

EP 4 752 768 A1

Server receives model parameter acquisition request sent by client, where model parameter acquisition request at least includes request authentication information — S20

Server performs identity authentication of client — S21

No → Authentication fails

Yes

Server generates key string based on historical optimized model parameters corresponding to client and performs encryption on current round of to-be sent optimized model parameters to generate ciphertext data packet and send ciphertext data packet to client — S22

Client determines decryption key based on identifier and decrypts received ciphertext data packet to obtain optimized model parameters — S23

Client updates local analysis model based on obtained optimized model parameters and sends local model parameters of updated local analysis model and loss function value to server, enabling server to determine a next round of optimized model parameters for client — S24

FIG. 3

## Description

### TECHNICAL FIELD

The present disclosure relates to the technical field of medical data, and specifically, to an encrypted transmission system and method for medical data.

### BACKGROUND

With the development of technology, the medical field increasingly employs digital technologies to construct medical platforms. However, since most of the medical data involves personal privacy, medical platforms often employ a federated learning mechanism to iteratively generate AI models for medical data. However, during data transmission within medical platforms, if a fixed encryption algorithm is used for encryption, frequent public disclosure of keys between the server and the client is required. This step is not only susceptible to being compromised but also consumes substantial computational resources.

### SUMMARY

An objective of embodiments of the present disclosure is to provide an encrypted transmission system and method for medical data, so as to improve the stability and security of medical data transmission and reduce a data transmission amount.

According to a first aspect, the present disclosure provides an encrypted transmission system for medical data. The encrypted transmission system includes a server and a plurality of clients, where each client is deployed at a different medical institution; the server, in response to a medical data analysis request, performs encrypted transmission of model parameters with each client by the following steps, so as to generate an optimized analysis model for a client issuing the medical data analysis request; the optimized analysis model is configured to analyze a medical data type and a rare disease type indicated by the medical data analysis request:

the server receives a model parameter acquisition request sent by the client, and the model parameter acquisition request at least includes request authentication information; the server performs identity authentication of the client based on the request authentication information corresponding to the client; in a case where the identity authentication of the client is passed, the server generates a key string based on historical optimized model parameters corresponding to the client and performs encryption on a current round of to-be sent optimized model parameters to generate a ciphertext data packet and send the ciphertext data packet to the client, and the ciphertext data packet at least includes ciphertext data and an identifier of the historical optimized model parameters used to generate the key string; the client determines a decryption key based on the identifier and decrypts the received ciphertext data packet to obtain the optimized model parameters; and the client updates a local analysis model based on the obtained optimized model parameters and sends local model parameters of the updated local analysis model and a loss function value to the server, enabling the server to determine a next round of optimized model parameters for the client, and the local analysis model is generated by the client through training of local medical data of the client based on an initial analysis model sent by the server.

In an optional embodiment, each model parameter is represented by a parameter name field and a parameter value field which are associated with each other, and the server converts the historical optimized model parameters into a first-length key string by the following manners: for each model parameter, converting the parameter name field into a first English character based on a first mapping rule, and converting the parameter value field into a second English character based on a second mapping rule; for each model parameter, concatenating the first English character and the second English character corresponding to the model parameter into a first English string; sequentially concatenating all first English strings corresponding to the model parameters to generate a second-length English string; and converting the second-length English string into the first-length key string.

In an optional embodiment, the server encrypts a current round of optimized model parameters to generate ciphertext data by the following manners: converting the current round of to-be-sent optimized model parameters into a first-length to-be-encrypted string; splitting the first-length to-be-encrypted string into a plurality of third-length to-be-encrypted character segments; performing an XOR operation between a first to-be-encrypted character segment and an initial vector character segment, and encrypting an XOR result using an SM4 encryption algorithm to generate a ciphertext block corresponding to the first to-be-encrypted character segment; for each remaining to-be-encrypted character segment other than the first to-be-encrypted character segment, performing an XOR operation between the remaining to-be-encrypted character segment and a ciphertext block corresponding to a previous to-be-encrypted character segment, and encrypting an XOR result using an SM4 encryption algorithm to generate a ciphertext block corresponding to the remaining to-be-encrypted character segment; and concatenating all ciphertext blocks sequentially to generate the ciphertext data.

In an optional embodiment, before performing each XOR operation, the server further replaces each English character in the to-be-encrypted character segment by the following manners: based on a position of a to-be-replaced English character in the to-be-encrypted string, locating a first target English character at the same position in the key string; based on the first target English character, determining a target displacement distance corresponding to the first target English character; and replacing the to-be-replaced English character with a second target English character, where a distance between the second target English character and the to-be-replaced English character in an English alphabet sequence is the target displacement distance.

In an optional embodiment, the model parameter acquisition request at least includes a request string, a first identifier value, and a second identifier value, and the server performs identity authentication for each client by the following manners: determining whether the first identifier value and the second identifier value are within an identifier-value range; in a case where both the first identifier value and the second identifier value are within the identifier-value range, calculating a third identifier value based on the request string; determining whether the first identifier value is equal to the third identifier value; and in a case where the first identifier value is equal to the third identifier value, determining that identity authentication of the client is passed.

In an optional embodiment, the server calculates the third identifier value R by the following manners:

$$R=(e+x_1) \bmod n;$$

$$e=\mathrm{Hash}\,(M');$$

$$M' = Za||M;$$

$$(x_1,\ y_1)=[s]G+[t]\mathrm{Pub}\,(x_0,\ y_0);$$

$$t=(r+s) \bmod n;$$

where $M$ is the request string, r is the first identifier value, s is the second identifier value, and Pub $(x_0, y_0)$ is an encryption public key.

In an optional embodiment, the first mapping rule is configured to indicate a relationship between the parameter name field and an English character, and the server is specifically configured to determine a first English character associated with the parameter name field in the first mapping rule.

In an optional embodiment, the server is specifically configured to calculate a product of a parameter value of the model parameter and a preset value, the product being rounded, and determine a second English character corresponding to the product based on a Base64 index table.

In an optional embodiment, the client determines historical optimized model parameters from decrypted optimized model parameters based on the identifier, and determines a first-length key string based on the historical optimized model parameters.

According to a second aspect, the present disclosure provides an encrypted transmission method for medical data. Each client is deployed in a different medical institution.

A server, in response to a medical data analysis request, performs encrypted transmission of model parameters with each client by the following steps, so as to generate an optimized analysis model for a client issuing the medical data analysis request, and the optimized analysis model is configured to analyze a medical data type and a rare disease type indicated by the medical data analysis request. The encrypted transmission method include:

responding, by the server, to a model parameter acquisition request sent by the client, wherein the model parameter acquisition request at least includes request authentication information;

performing, by the server, identity authentication of the client based on the request authentication information corresponding to the client;

in a case where the identity authentication of the client is passed, generating, by the server, a key string based on historical optimized model parameters corresponding to the client and performing encryption on a current round of to-be sent optimized model parameters to generate a ciphertext data packet and sending the ciphertext data packet to the client, wherein the ciphertext data packet at least includes ciphertext data and an identifier of the historical optimized model parameters used to generate the key string;

determining, by the client, a decryption key based on the identifier and decrypting the received ciphertext data packet to obtain the optimized model parameters; and

updating, by the client, a local analysis model based on the obtained optimized model parameters and sending local model parameters of the updated local analysis model and a loss function value to the server, enabling the server to determine a next round of optimized model parameters for the client, wherein the local analysis model is generated by the client through training of local medical data of the client based on an initial analysis model sent by the server.

The present disclosure provides an encrypted transmission system and method for medical data. A server and clients perform model training based on a federated learning mechanism. In a process of transmitting model parameters between the server and the clients, an SM2 signature verification method and an SM4 CBC-mode encryption method are combined, and historical transmitted model parameters are used as an encryption key of the SM4 CBC mode. Therefore, the stability and security of medical data transmission are ensured, and a data transmission amount is further reduced.

## BRIEF DESCRIPTION OF THE DRAWINGS

To more clearly illustrate the technical solutions of the embodiments of the present disclosure, the drawings required in the embodiments of the present disclosure will be briefly described below. It should be understood that the following drawings only illustrate some embodiments of the present disclosure and therefore should not be considered as limitations of the scope. For those of ordinary skill in the art, other related drawings can be obtained according to these drawings without inventive efforts.

FIG. 1 is a schematic diagram of a structure of a medical data processing system according to an embodiment of the present disclosure;

FIG. 2 is a flowchart of a medical data processing method according to an embodiment of the present disclosure;

FIG. 3 is a flowchart of an encrypted transmission method for medical data according to an embodiment of the present disclosure; and

FIG. 4 is a schematic diagram of a structure of an encrypted transmission system for medical data according to an embodiment of the present disclosure.

## DETAILED DESCRIPTION OF THE EMBODIMENTS

First, an applicable scenario of the present disclosure is described. A technical solution of the present disclosure is applicable to a medical data processing system, and the medical data processing system is specifically a medical data processing system constructed based on a federated learning mechanism. The processing system includes a server and a plurality of clients, and each client is deployed in a different medical institution.

A conventional federated learning model training mechanism is as follows: a model is issued by the server to the clients of the medical institutions for local training, and then models uploaded by the clients are aggregated and evaluated. During model training between the server and the clients, frequent data transmission is required, which occupies a large amount of computing resources and increases a possibility of attacks.

Therefore, the present disclosure provides an encrypted transmission system and method for medical data, which improves the stability and security of medical data transmission and reduces a data transmission amount.

Technical solutions in embodiments of the present disclosure are described below with reference to drawings in embodiments of the present disclosure.

Embodiment 1

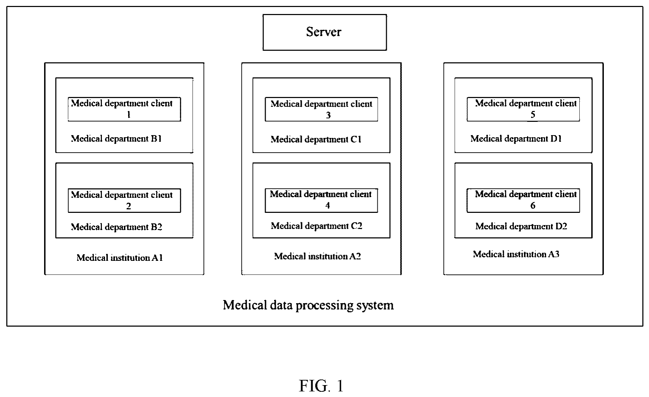

FIG. 1 is a schematic diagram of a structure of a medical data processing system according to an embodiment of the present disclosure. As shown in FIG. 1, the encrypted transmission system (which can also be a medical data processing system) includes a server and a plurality of clients, where each client is deployed at a different medical institution. The server responds to a medical data analysis request, and generates an optimized analysis model for a client issuing the medical data analysis request. The optimized analysis model is configured to analyze a medical data type and a rare disease type indicated by the medical data analysis request.

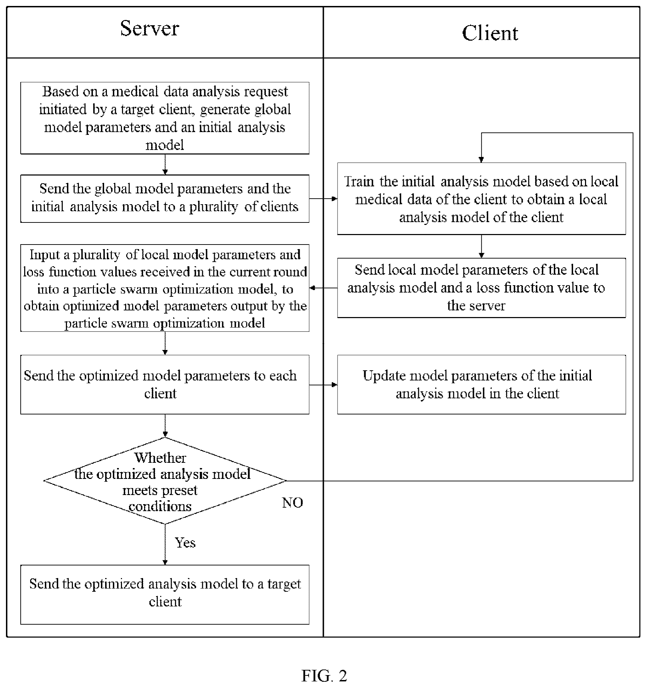

Further, as shown in FIG. 2, the model training between the server and the clients can be completed by the following steps:

Step 1. The server, based on a medical data analysis request initiated by a target client, generates global model parameters and an initial analysis model, and sends the global model parameters and the initial analysis model to a plurality of clients.

The target client refers to a target medical institution issuing the medical data analysis request. After the medical data analysis request is sent to the server, the server broadcasts the request to clients corresponding to remaining medical institutions.

The medical data analysis request is configured to indicate a medical data type and a rare disease type to be analyzed by the target medical institution corresponding to the target client. The plurality of clients include the target client and other clients participating in the medical data analysis request.

The remaining medical institutions can autonomously determine whether to respond to the medical data analysis request, and assist the target medical institution in jointly training an AI model based on local medical data.

The medical data type can include medical images, case forms, medical records in text, and gene sequences. The rare disease type can be classified into a plurality of systems, such as hematologic system diseases, nervous system diseases, cardiovascular system diseases, urinary system diseases, endocrine system diseases, skeletal system diseases, respiratory system diseases, immune system diseases, digestive system diseases, ophthalmologic system diseases, dermatologic system diseases, pediatric system diseases, and obstetric-gynecologic system diseases. Each system includes at least one disease. For example, the respiratory system diseases specifically include lymphangioleiomyomatosis, pulmonary alveolar proteinosis, and idiopathic pulmonary fibrosis.

For example, a target medical institution A initiates a medical data analysis request. The medical data analysis request indicates that the medical data type to be analyzed is medical imaging, specifically coronary CTA images, and the rare disease type to be analyzed can be coronary artery ectasia. That is, an AI model to be constructed by the target medical institution A can have an input including patient coronary CTA images, and an output including a probability of disease, a disease severity level, or identification of key diseased regions, and other analysis results.

The remaining medical institutions can, based on local medical data, determine whether to join the medical data analysis request of the target medical institution A.

Subsequently, the server can determine the target medical institution A and other medical institutions B, C, D, and E that join the medical data analysis request, and send global model parameters and the initial analysis model to clients corresponding to medical institutions A, B, C, D, and E.

The server can match a suitable AI model and corresponding global model parameters based on the medical data analysis request indicating the medical data type and the rare disease type to be analyzed by the target medical institution. For example, in a case where the medical data type is a medical imaging category, a convolutional neural network (CNN) can be selected as the initial analysis model; and in a case where the medical data type is time-series data such as electrocardiogram, a recurrent neural network (RNN) can be selected as the initial analysis model. The global model parameters can include initial values commonly used for the model set based on experience.

Step 2. For each client, the initial analysis model is trained based on local medical data of the client to obtain a local analysis model of the client, and local model parameters of the local analysis model and a loss function value are sent to the server. The local medical data are matched with the medical data type and the rare disease type.

For the clients of different medical institutions A, B, C, D, and E, each client begins training the initial analysis model based on the local medical data. Training manners, including a loss function, iteration number, and other training parameters, can be uniformly specified by the server, for example, 5 or 10 iterations per round for a total of 10 rounds. For example, medical institution A inputs coronary CTA images of patient a into the initial analysis model, and training enables the model to output a probability value of disease for the patient a. The medical institution B can input coronary CTA images of patient b into the initial analysis model, and training enables the model to output a probability value of disease for the patient b. By analogy, the local analysis models are obtained.

After completion of each round of iteration, each client can feedback local model parameters of the current local analysis model and a loss function value to the server, which can be represented as

$$S_A = \{s_A^1, \ s_A^2, \ s_A^3, \ s_A^4, \ Lost_A\} \quad ,$$

$S_B = \{s_B^1, \ s_B^2, \ s_B^3, \ s_B^4, \ Lost_B\}, \ \dots \ , \ S_E = \{s_E^1, \ s_E^2, \ s_E^3, \ s_E^4, \ Lost_E\}$, where $s^1$, $s^2$, $s^3$, and $s^4$ respectively represent a plurality of different sub-parameter items of the local analysis model, and *Lost* represents the loss function value.

Step 3. The server inputs a plurality of local model parameters and loss function values received in the current round into a particle swarm optimization model to obtain optimized model parameters output by the particle swarm optimization model, and sends the optimized model parameters to each client to update model parameters of the initial analysis model in each client.

The $S_A$-$S_E$ received by the server in each round are input into the particle swarm optimization model. As shown in FIG. 2, the particle swarm optimization model can output optimized model parameters of each round by the following manners:

S100. The local model parameter sent by each client in the current round is used as a position of particle i, and a loss function difference of each client is used as a velocity of particle i.

The loss function difference is a difference between the loss function value sent by the client in the current round and a mean of loss function values of the previous round.

For each particle i, the position of this particle can be represented as

$$X_A = \{s_A^1,\ s_A^2,\ s_A^3,\ s_A^4\},\ X_B = \{s_B^1,\ s_B^2,\ s_B^3,\ s_B^4\}.\ ...,\ X_E = \{s_E^1,\ s_E^2,\ s_E^3,\ s_E^4\}.$$

For each particle i, the velocity of this particle can be represented as $V_A = \overline{Lost'} - Lost_A$, $V_B = \overline{Lost'} - Lost_B$, ..., $V_E = \overline{Lost'} - Lost_E$. where $\overline{Lost'}$ is a mean of loss function values corresponding to all clients in the previous round.

S101. Whether a convergence condition is met is determined.

The convergence condition can be the number of iterations.

A fitness value of each particle is calculated through an objective function and compared with a best position of the particle to update the best position of the particle.

S102. If the convergence condition is not met, the position and velocity of each particle i are updated, and execution returns to the step of determining whether the convergence condition is met.

In the step S102, the position and velocity of each particle i can be updated by the following manners:

$$V_i' = V_i + C_1 r_1\ (pbest_i - X_i)\ + C_2 r_2 (gbest_i - X_i);$$

$$X_i' = X_i + V_i;$$

where $V_i'$ is the velocity of particle i after update, $V_i$ is the velocity of particle i before update, $X_i'$ is the position of particle i after update, $X_i$ is the position of particle i before update, $pbest_i$ is the best position of particle i, $gbest_i$ is a global best position, $C_1$ and $C_2$ are learning parameter values (generally $C_1 = C_2 = 2$), $r_1$ and $r_2$ are random parameter values in [0,1], and i = 1, 2, 3 ... N, where N is a total number of particles.

S103. If the convergence condition is met, the best position and the global best position of each particle i are outputted as optimized model parameters.

Finally, in a case where the convergence condition is met, the best position and the global best position of each particle i are obtained.

Step 4. The server determines an optimized analysis model based on the optimized model parameters, and determines whether the optimized analysis model meets preset conditions. If the optimized analysis model does meet preset conditions, execution returns to Step 2; if the optimized analysis model meets preset conditions, the optimized analysis model is sent to the target client.

The server can use the optimized model parameters as model parameters of the initial analysis model to determine the optimized analysis model. The accuracy of the optimized analysis model can then be calculated. In a case where the accuracy meets requirements, iteration can end, and the obtained optimized analysis model can be sent to the target client. In a case where the accuracy does not meet requirements, the optimized model parameters are fed back to each client to continue iterative training.

Step 5. The target client inputs to-be-tested medical data matching the medical data type into the optimized analysis model to obtain analysis results corresponding to the rare disease type from the optimized analysis model.

The target client can obtain corresponding analysis results through the final optimized analysis model. The optimized analysis model has higher accuracy and better generalization capability, thereby providing improved assistance in rare disease analysis.

The medical data processing system provided by the present disclosure, by combining a particle swarm optimization algorithm with a federated learning-based medical data processing system, balances the impact of data differences among different clients on model training, is conducive to global model integration, and thus can improve the accuracy and generalization capability of constructing rare disease analysis models.

Embodiment 2

For the aforementioned medical data processing system, to further improve the security of medical data, especially data of rare disease patients, during the data transmission between the server and clients, the server can be limited to interact with the Internet only through port 8443. The server can also be provided with security protections such as a firewall and a whitelist.

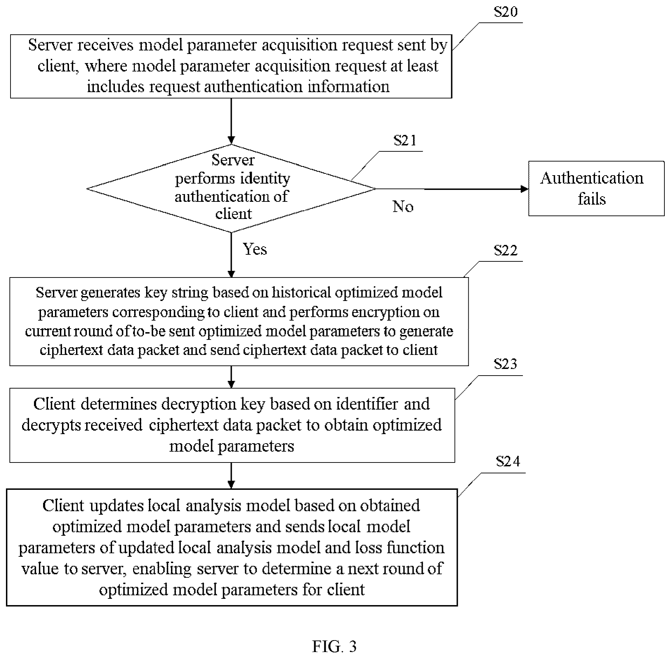

As shown in FIG. 3, sensitive data can be transmitted through encrypted transmission. Taking model parameters as an example, a data transmission process between the server and clients is described as follows:

S20. The server receives a model parameter acquisition request sent by the client, and the model parameter acquisition request at least includes request authentication information.

After completion of local training, each client can send the model parameter acquisition request to the server, indicating that the client is ready for a next training round. The model parameter acquisition request at least includes a request string, a first identifier value, and a second identifier value. The request string represents actual string data of the model parameter acquisition request and can include a client ID and other addresses or instructions. The first identifier value and the second identifier value constitute a signature of the model parameter acquisition request, which are used by the server to perform signature verification of the client.

S21. The server performs identity authentication of the client based on the request authentication information corresponding to the client.

Specifically, the identity authentication is implemented based on the SM2 algorithm. The server can perform identity authentication for each client by the following manners:

Whether the first identifier value r and the second identifier value s are within an identifier value range [1, n-1] is determined, where n is an order of the elliptic curve base point $(xG, yG)$.

$t=(r+s) \bmod n$ is calculated. If t=0, the authentication fails.

If both values are within the identifier value range, a third identifier value is calculated based on the request string.

The server can calculate the third identifier value R by the following manners:

$$R=(e+x_1) \bmod n;$$

$$e=\text{Hash}(M');$$

$$M' = Za||M;$$

$$(x_1, y_1)=[s]G+[t]\text{Pub}(x_0, y_0);$$

$$t= (r+s) \bmod n;$$

where $M$ is the request string, r is the first identifier value, s is the second identifier value, and Pub $(x_0, y_0)$ is an encryption public key, and e needs to be rounded.

The [s]G represents a point obtained by multiplying the elliptic curve base point G by s on a preset elliptic curve, and [t]Pub $(x_0, y_0)$ represents a point obtained by multiplying a point corresponding to the encryption public key PA by t on the preset elliptic curve.

Client A possesses a distinguishable identifier IDA having a length of entlen bits, a private key dA, and a public key PA= (xA, yA) =Pub $(x_0, y_0)$. The server possesses a private key dB having a length of entlen bits and a public key PB= (xB, yB). A hash value Za having a length of 256 bits can be calculated using the SM3 hash algorithm according to the following formula:

$$Za = H256 \ (entlen||IDA||a||b||xG||yG||xA||yA) ;$$

where a and b are parameters of a preset elliptic curve equation, and ‖ represents concatenation between strings.

Whether the first identifier value is equal to the third identifier value is determined. If the first identifier value is equal to the third identifier value, the identity authentication of the client is passed; and if the first identifier value is not equal to the third identifier value, the authentication fails.

S22. In a case where the identity authentication of the client is passed, the server generates a key string based on historical optimized model parameters corresponding to the client and performs encryption on a current round of to-be sent optimized model parameters to generate a ciphertext data packet and send the ciphertext data packet to the client, and the ciphertext data packet at least includes ciphertext data and an identifier of the historical optimized model parameters used to generate the key string.

In the step S22, the SM4 CBC model can be used to encrypt the optimized model parameters. During the first round of iteration training, a preset key string can be used. In subsequent iteration training, a key string can be generated by randomly selecting historical optimized model parameters.

It can be understood that each model parameter can be represented by a parameter name field and a parameter value field which are associated with each other. For example, a model parameter can be [lr, 0.1], [n_epoch, 10], and so on.

The server can convert historical optimized model parameters into a first-length key string by the following manners.

For each model parameter, the parameter name field is converted into a first English character based on a first mapping rule, and the parameter value field is converted into a second English character based on a second mapping rule.

The first mapping rule is configured to indicate a relationship between the parameter name field and an English character, and the server is specifically configured to determine a first English character associated with the parameter name field in the first mapping rule.

The first mapping rule can take the form of a mapping table. Each model parameter corresponds to an English character. For example, the parameter lr is associated with the English character A, and the parameter n_epoch is associated with the English character B, and so on.

The second mapping rule can be implemented using the Base64 encoding method. The server converts the corresponding parameter value into the second English character. Specifically, the server can calculate a product of the parameter value of the model parameter and a preset value, and the product is rounded. Based on a Base64 index table, a second English character corresponding to the product is determined.

For example, for a parameter value field of 0.1, multiplied by 16, the result is 2. The English character C corresponding to 2 serves as the second English character. For a parameter value field of 10, multiplied by 16, the result is 160, which can correspond to 1-B, 6-G, 0-A, forming BGA as the second English character.

Other encoding methods can also be employed to convert the parameter value field into English characters, without limitation.

For each model parameter, the first English character and the second English character corresponding to the model parameter are concatenated to form a first English string.

For example, [lr, 0.1] and [n_epoch, 10] can be concatenated into corresponding first English strings "AC" and "BBGA", respectively.

All first English strings corresponding to the model parameters are sequentially concatenated to generate a second-length English string.

For instance, "AC" and "BBGA" are concatenated as "ACBBGA".

The second-length English string is then converted into a first-length key string.

Since the key string is required to be 16 characters in length (i.e., 128 bits), if the final concatenated English string has a second length less than 16, padding can be applied. If the final concatenated English string has a second length exceeding 16, truncation can be applied. It should be noted that the conversion method needs to be synchronized with the client. For example, if the final concatenated English string exceeds 16 characters, 16 English characters can be truncated starting from the third character. A corresponding identifier indicating the starting position of the English character truncation can be included in the transmitted ciphertext data packet.

After the key string is determined, the server can perform encryption on a current round of to-be-sent optimized model parameters to generate ciphertext data by the following manners.

The current round of to-be-sent optimized model parameters is converted into a first-length to-be-encrypted string.

The optimized model parameters are converted into English characters and split into a 128-bit to-be-encrypted string. Each to-be-encrypted string is processed in the same manner.

The first-length to-be-encrypted string is split into a plurality of third-length to-be-encrypted character segments.

Each 128-bit to-be-encrypted string is averagely split into four 32-bit to-be-encrypted character segments, referred to as to-be-encrypted character segment 1, to-be-encrypted character segment 2, to-be-encrypted character segment 3, and to-be-encrypted character segment 4.

The first to-be-encrypted character segment and an initial vector character segment are subjected to an XOR operation, and the XOR result is encrypted by the SM4 encryption algorithm to generate a ciphertext block corresponding to the first to-be-encrypted character segment.

The initial vector character segment can be preset or randomly generated. The initial vector character segment has a length equal to the to-be-encrypted character segment. Firstly, the to-be-encrypted character segment 1 and the initial vector character segment are subjected to an XOR operation, and the XOR result is encrypted by the SM4 algorithm to generate a ciphertext block 1.

For each remaining to-be-encrypted character segment other than the first to-be-encrypted character segment, the remaining to-be-encrypted character segment is subjected to an XOR operation with a ciphertext block corresponding to a previous to-be-encrypted character segment, and the XOR result is encrypted through an SM4 encryption algorithm to generate a ciphertext block corresponding to the remaining to-be-encrypted character segment.

Subsequently, the ciphertext block 1 is subjected to an XOR operation with the to-be-encrypted character segment 2, and the XOR result is encrypted through the SM4 encryption algorithm to generate a ciphertext block 2. By analogy, the ciphertext block 1, the ciphertext block 2, the ciphertext block 3 and the ciphertext block 4 can be obtained finally.

All ciphertext blocks are concatenated in sequence to generate ciphertext data.

Through concatenation of the ciphertext block 1, the ciphertext block 2, the ciphertext block 3, and the ciphertext block 4, the corresponding ciphertext data is obtained.

It can be understood the number of ciphertext data and the number of to-be-encrypted strings are in one-to-one correspondence.

S23. The client determines a decryption key based on the identifier and decrypts the received ciphertext data packet to obtain the optimized model parameters.

Unlike the prior art, the server does not transmit model parameters used for generating a key to the client, but transmits an identifier used for indicating historical optimized model parameters for generating a key string, which greatly reduces a data transmission amount between the server and the client. Moreover, the historical optimized model parameters exist at the client locally and do not undergo network transmission, which avoids attacks and prevents leakage of medical data.

The identifier can be the iteration round and the generation time corresponding to the historical optimized model. The client determines historical optimized model parameters from decrypted optimized model parameters based on the identifier, and determines a first-length key string based on the historical optimized model parameters.

The client determines historical optimized model parameters used by the server for generating an encryption key string based on the identifier and generates a corresponding decryption key string through a manner similar to the server. Then, the client decrypts the ciphertext data packet to obtain the current round of optimized model parameters.

S24. The client updates a local analysis model based on the obtained optimized model parameters and sends local model parameters of the updated local analysis model and a loss function value to the server, enabling the server to determine a next round of optimized model parameters for the client, and the local analysis model is generated by the client through training of local medical data of the client based on an initial analysis model sent by the server.

Embodiment 3

In an embodiment of the present disclosure, to further improve the security of encrypted transmission of medical data, the present disclosure proposes an improved SM4 CBC algorithm, which specifically includes the following steps. Before performing each XOR operation, the server further replaces each English character in the to-be-encrypted character segment by the following manners.

Based on a position of a to-be-replaced English character in the to-be-encrypted string, a first target English character at the same position in the key string is located.

For example, the to-be-encrypted character segment 2 of the to-be-encrypted string can be "DHAC". Taking "D" as an example, a position sequence is 5, and a first target English character at a same position sequence in the key string is "G".

Based on the first target English character, a target displacement distance corresponding to the first target English character is determined.

"G" is a seventh character in an English alphabet sequence, and therefore the target displacement distance is 7. The to-be-replaced English character is replaced with a second target English character, where a distance between the second target English character and the to-be-replaced English character in an English alphabet sequence is the target displacement distance.

In the English alphabet sequence, a letter spaced 7 characters from "D" is "L", which is the second target English character. Subsequently, "H" is replaced with "J", "A" is replaced with "C", and "C" is replaced with "E". Thus, "DHAC" is replaced with "LJCE".

Therefore, before each to-be-encrypted character segment is subjected to the XOR operation, an irregular replacement is performed, and a replacement rule is determined in the key generated by the model parameters, thereby increasing difficulty of cracking a ciphertext data packet and improving the security of encrypted transmission of rare-disease data.

Embodiment 4

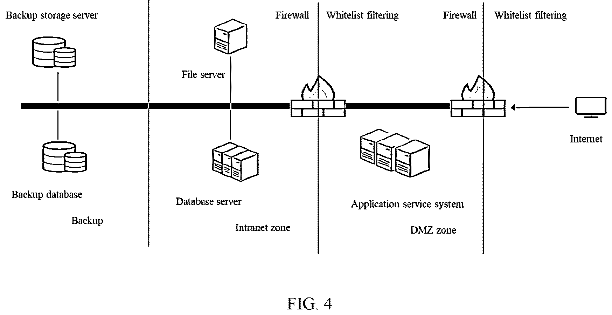

As shown in FIG. 4, the present disclosure provides a medical data processing system (which can also be an encrypted transmission system). The network architecture can include an application service system, a file server, a database server, a backup database, and a backup storage server. The application service system can serve as the server in the foregoing embodiments, and is configured to execute steps such as data transmission and iterative model training.

For different clients or users, different access permissions can be granted for different modules or functions. A strategy for preventing replay attacks is also designed to prevent an attacker from replaying intercepted network requests. Meanwhile, data transmitted from the client (such as a medical data analysis request or a model parameter acquisition request) can be filtered to avoid malicious users obtaining sensitive information through SQL injection attacks.

Embodiment 5

Based on the same inventive concept, an embodiment of the present disclosure further provides an encrypted transmission method for medical data. Each client is deployed in a different medical institution.
A server, in response to a medical data analysis request, performs encrypted transmission of model parameters with each client by the following steps, so as to generate an optimized analysis model for a client issuing the medical data analysis request; the optimized analysis model is configured to analyze a medical data type and a rare disease type indicated by the medical data analysis request. The method includes the following steps:

responding, by the server, to a model parameter acquisition request sent by the client, wherein the model parameter acquisition at least includes request authentication information;

performing, by the server, identity authentication of the client based on the request authentication information corresponding to the client;

in a case where the identity authentication of the client is passed, generating, by the server, a key string based on historical optimized model parameters corresponding to the client and performing encryption on a current round of to-be sent optimized model parameters to generate a ciphertext data packet and sending the ciphertext data packet to the client, wherein the ciphertext data packet at least includes ciphertext data and an identifier of the historical optimized model parameters used to generate the key string;

determining, by the client, a decryption key based on the identifier and decrypting the received ciphertext data packet to obtain the optimized model parameters; and

updating, by the client, a local analysis model based on the obtained optimized model parameters and sending local model parameters of the updated local analysis model and a loss function value to the server, enabling the server to determine a next round of optimized model parameters for the client, wherein the local analysis model is generated by the client through training of local medical data of the client based on an initial analysis model sent by the server.

In the embodiments provided in the present disclosure, it should be understood that the disclosed device and method can be implemented in another manner. The described device embodiments are merely examples. For example, the division of units is merely logical function division and can be other division in actual implementation. For example, a plurality of units or components can be combined or integrated into another system, or some features can be ignored or cannot be performed. In addition, the displayed or discussed mutual couplings or direct couplings or communications connections can be implemented by using some communication interfaces. The indirect couplings or communications connections between apparatuses or units can be implemented in electrical, mechanical, or other forms.
In addition, units described as separate parts can or cannot be physically separate. Parts displayed as units can or cannot be physical units, to be specific, can be located in one position, or can be distributed on a plurality of network units. Some or all of the units can be selected based on actual requirements to achieve the objectives of the solutions of embodiments.
In addition, the functional modules in the embodiments of the present disclosure can be integrated together to form an independent part, or each module can exist independently, or two or more modules can be integrated to form an independent part.
It should be understood that when the functions are implemented in a form of a software functional module and sold or used as an independent product, the functions can be stored in a computer-readable storage medium. Based on such an understanding, the technical solutions of the present disclosure essentially or parts contributing to the prior art or some of the technical solutions can be embodied in a form of a software product. The computer software product is stored in a storage medium, and includes several instructions for instructing a computer device (which can be a personal computer, a server, a network device, or the like) to perform all or some of the steps of the methods described in the embodiments of the present disclosure. The foregoing storage medium includes any medium that can store program code, such as a USB flash drive, a removable hard disk, a read-only memory (ROM), a random access memory (RAM), a magnetic disk, or an optical disc.
In the present disclosure, relational terms such as "first" and "second" are merely used for distinguishing one entity or operation from another entity or operation, and are not necessarily required to indicate or imply any actual relationship or sequence between the entities or operations.

The above description is merely embodiments of the present disclosure and is not intended to limit the scope of protection of the present disclosure. Various modifications and variations can be made by those skilled in the art. Any modification, equivalent replacement, improvement, or the like made without departing from the spirit and principle of the present disclosure shall fall within the protection scope of the present disclosure.

**Claims**

1. An encrypted transmission system for medical data, **characterized by** comprising a server and a plurality of clients, wherein each client is deployed in a different medical institution;

   the server, in response to a medical data analysis request, performs encrypted transmission of model parameters with each client by following steps, so as to generate an optimized analysis model for a client issuing the medical data analysis request, and the optimized analysis model is configured to analyze a medical data type and a rare disease type indicated by the medical data analysis request; and the steps comprise
   receiving, by the server, a model parameter acquisition request sent by the client, wherein the model parameter acquisition request at least comprises request authentication information;
   performing, by the server, identity authentication of the client based on the request authentication information corresponding to the client;
   generating, in a case where the identity authentication of the client is passed, a key string by the server based on historical optimized model parameters corresponding to the client, performing encryption on a current round of to-be sent optimized model parameters to generate a ciphertext data packet, and sending the ciphertext data packet to the client, wherein the ciphertext data packet at least comprises ciphertext data and an identifier of the historical optimized model parameters used to generate the key string;
   determining, by the client, a decryption key based on the identifier and decrypting the received ciphertext data packet to obtain the optimized model parameters; and
   updating, by the client, a local analysis model based on the obtained optimized model parameters, sending local model parameters of the updated local analysis model and a loss function value to the server, and enabling the server to determine a next round of optimized model parameters for the client, wherein the local analysis model is generated by the client through training of local medical data of the client based on an initial analysis model sent by the server.

2. The system according to claim 1, wherein each model parameter is represented by a parameter name field and a parameter value field which are associated with each other, and the server converts the historical optimized model parameters into a first-length key string by the following manners:

   converting, for each model parameter, the parameter name field into a first English character based on a first mapping rule, and converting the parameter value field into a second English character based on a second mapping rule;
   concatenating, for each model parameter, the first English character and the second English character corresponding to the model parameter into a first English string;
   sequentially concatenating all first English strings corresponding to the model parameters to generate a second-length English string; and
   converting the second-length English string into the first-length key string.

3. The system according to claim 2, the server performs encryption on a current round of to-be-sent optimized model parameters to generate ciphertext data by following manners:

   converting the current round of to-be-sent optimized model parameters into a first-length to-be-encrypted string;
   splitting the first-length to-be-encrypted string into a plurality of third-length to-be-encrypted character segments;
   performing an XOR operation between a first to-be-encrypted character segment and an initial vector character segment, and encrypting an XOR result using an SM4 encryption algorithm to generate a ciphertext block corresponding to the first to-be-encrypted character segment;
   performing, for each remaining to-be-encrypted character segment other than the first to-be-encrypted character segment, an XOR operation between the remaining to-be-encrypted character segment and a ciphertext block corresponding to a previous to-be-encrypted character segment, and encrypting an XOR result using an SM4 encryption algorithm to generate a ciphertext block corresponding to the remaining to-be-encrypted character segment; and
concatenating all ciphertext blocks sequentially to generate the ciphertext data.

4. The system according to claim 3, wherein before performing each XOR operation, the server further replaces each English character in the to-be-encrypted character segment by following manners:

locating, based on a position of a to-be-replaced English character in the to-be-encrypted string, a first target English character at the same position in the key string;
determining, based on the first target English character, a target displacement distance corresponding to the first target English character; and
replacing the to-be-replaced English character with a second target English character, wherein a distance between the second target English character and the to-be-replaced English character in an English alphabet sequence is the target displacement distance.

5. The system according to claim 1, wherein the model parameter acquisition request at least comprises a request string, a first identifier value, and a second identifier value, and the server performs identity authentication for each client by following manners:

determining whether the first identifier value and the second identifier value are within an identifier-value range;
calculating, in a case where both the first identifier value and the second identifier value are within the identifier-value range, a third identifier value based on the request string;
determining whether the first identifier value is equal to the third identifier value; and
determining, in a case where the first identifier value is equal to the third identifier value, that identity authentication of the client is passed.

6. The system according to claim 5, wherein the server calculates the third identifier value R by following manners:

$$R=(e+x_1) \bmod n;$$

$$e=Hash\ (M');$$

$$M' = Za||M;$$

$$(x_1,\ y_1)=[s]G+[t]Pub\ (x_0,\ y_0);$$

$$t=(r+s) \bmod n;$$

where $M$ is the request string, r is the first identifier value, s is the second identifier value, and Pub $(x_0, y_0)$ is an encryption public key.

7. The system according to claim 2, wherein the first mapping rule is configured to indicate a relationship between the parameter name field and an English character, and the server is specifically configured to determine a first English character associated with the parameter name field in the first mapping rule.

8. The system according to claim 2, wherein the server is specifically configured to calculate a product of a parameter value of the model parameter and a preset value, the product is rounded, and
determine a second English character corresponding to the product based on a Base64 index table.

9. The system according to claim 1, wherein the client determines the historical optimized model parameters from decrypted optimized model parameters based on the identifier, and determines a first-length key string based on the historical optimized model parameters.

10. An encrypted transmission method for medical data, **characterized in that** each client is deployed in a different medical institution,

a server, in response to a medical data analysis request, performs encrypted transmission of model parameters with each client by following steps, so as to generate an optimized analysis model for a client issuing the medical data analysis request, wherein the optimized analysis model is configured to analyze a medical data type and a rare disease type indicated by the medical data analysis request; and the steps comprise:

responding, by the server, to a model parameter acquisition request sent by the client, wherein the model parameter acquisition request at least comprises request authentication information;

performing, by the server, identity authentication of the client based on the request authentication information corresponding to the client;

generating, in a case where the identity authentication of the client is passed, a key string by the server based on historical optimized model parameters corresponding to the client and performs encryption on a current round of to-be sent optimized model parameters to generate a ciphertext data packet and sending the ciphertext data packet to the client, wherein the ciphertext data packet at least comprises ciphertext data and an identifier of the historical optimized model parameters used to generate the key string;

determining, by the client, a decryption key based on the identifier and decrypting the received ciphertext data packet to obtain the optimized model parameters; and

updating, by the client, a local analysis model based on the obtained optimized model parameters, sending local model parameters of the updated local analysis model and a loss function value to the server, and enabling the server to determine a next round of optimized model parameters for the client, wherein the local analysis model is generated by the client through training of local medical data of the client based on an initial analysis model sent by the server.

Server

Medical department client 1

Medical department B1

Medical department client 2

Medical department B2

Medical institution A1

Medical department client 3

Medical department C1

Medical department client 4

Medical department C2

Medical institution A2

Medical department client 5

Medical department D1

Medical department client 6

Medical department D2

Medical institution A3

Medical data processing system

FIG. 1

| Server | Client |
|---|---|
| Based on a medical data analysis request initiated by a target client, generate global model parameters and an initial analysis model | |
| Send the global model parameters and the initial analysis model to a plurality of clients | Train the initial analysis model based on local medical data of the client to obtain a local analysis model of the client |
| Input a plurality of local model parameters and loss function values received in the current round into a particle swarm optimization model, to obtain optimized model parameters output by the particle swarm optimization model | Send local model parameters of the local analysis model and a loss function value to the server |
| Send the optimized model parameters to each client | Update model parameters of the initial analysis model in the client |
| Whether the optimized analysis model meets preset conditions  NO | |
| Yes | |
| Send the optimized analysis model to a target client | |

FIG. 2

S20

Server receives model parameter acquisition request sent by client, where model parameter acquisition request at least includes request authentication information

S21

Server performs identity authentication of client

No

Authentication fails

Yes

S22

Server generates key string based on historical optimized model parameters corresponding to client and performs encryption on current round of to-be sent optimized model parameters to generate ciphertext data packet and send ciphertext data packet to client

S23

Client determines decryption key based on identifier and decrypts received ciphertext data packet to obtain optimized model parameters

S24

Client updates local analysis model based on obtained optimized model parameters and sends local model parameters of updated local analysis model and loss function value to server, enabling server to determine a next round of optimized model parameters for client

FIG. 3

Backup storage server

File server

Firewall   Whitelist filtering   Firewall   Whitelist filtering

Backup database

Database server

Application service system

Internet

Backup

Intranet zone

DMZ zone

FIG. 4

**EUROPEAN SEARCH REPORT**

Application Number

EP 25 21 9981

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 116 484 426 A (UNIV CHONGQING POSTS & TELECOM) 25 July 2023 (2023-07-25) | 1-3,5-10 | INV.<br>G06F21/60 |
| A | * figure 1 *<br>* paragraph [0003] *<br>* paragraph [0023] - paragraph [0025] *<br>* paragraph [0031] - paragraph [0035] *<br>* paragraph [0039] *<br>----- | 4 | G06F21/62<br>G06N20/20<br>G16H50/20<br>H04L9/06<br>H04L9/08<br>H04L9/40 |
| A | CN 118 366 577 A (ZHEJIANG LAB) 19 July 2024 (2024-07-19)<br>* figure 2 *<br>* paragraph [0033] *<br>* paragraph [0037] - paragraph [0038] *<br>* paragraph [0082] *<br>----- | 1-10 | G16H10/60 |
| A | US 2023/290456 A1 (BAROR YUVAL [IL] ET AL) 14 September 2023 (2023-09-14)<br>* figures 10A-10B *<br>* paragraph [0007] *<br>* paragraph [0039] *<br>* paragraph [0042] *<br>* paragraph [0072] - paragraph [0075] *<br>----- | 1-10 | |

TECHNICAL FIELDS
SEARCHED (IPC)

G06F
H04L
G06E
G16H
G06N

The present search report has been drawn up for all claims

| Place of search | Date of completion of the search | Examiner |
|---|---|---|
| Munich | 25 February 2026 | Caragata, Daniel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 9981

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2026

| Patent document cited in search report | | | Publication date | Patent family member(s) | | Publication date |
|---|---|---|---|---|---|---|
| CN 116484426 | A | | 25-07-2023 | NONE | | |
| CN 118366577 | A | | 19-07-2024 | NONE | | |
| US 2023290456 | A1 | | 14-09-2023 | EP | 4490645 A1 | 15-01-2025 |
| | | | | IL | 315488 A | 01-11-2024 |
| | | | | KR | 20240154665 A | 25-10-2024 |
| | | | | US | 2023289728 A1 | 14-09-2023 |
| | | | | US | 2023290456 A1 | 14-09-2023 |
| | | | | WO | 2023172972 A1 | 14-09-2023 |

EPO FORM P0459

For more details about this annex : see Official Journal of the European Patent Office, No. 12/82